(19) Europäisches Patentamt
European Patent Office
Office européen des brevets

(11) **EP 4 010 500 B1**

(12) **EUROPÄISCHE PATENTSCHRIFT**

(45) Veröffentlichungstag und Bekanntmachung des Hinweises auf die Patenterteilung:
**19.02.2025 Patentblatt 2025/08**

(21) Anmeldenummer: **19765656.4**

(22) Anmeldetag: **05.08.2019**

(51) Internationale Patentklassifikation (IPC):
**C12R 1/19** (2006.01)   **C12P 21/00** (2006.01)
**C07K 14/245** (2006.01)

(52) Gemeinsame Patentklassifikation (CPC):
**C12N 9/50; C07K 16/2875; C12N 9/1074;
C12N 15/70; C12P 21/02; C12Y 204/01019;**
C07K 2317/10; C07K 2317/55; C07K 2319/02

(86) Internationale Anmeldenummer:
**PCT/EP2019/071006**

(87) Internationale Veröffentlichungsnummer:
**WO 2021/023367 (11.02.2021 Gazette 2021/06)**

(54) **BAKTERIENSTAMM ZUR FREISETZUNG EINES REKOMBINANTEN PROTEINS IN EINEM FERMENTATIONSVERFAHREN**

BACTERIAL STRAIN FOR RELEASING A RECOMBINANT PROTEIN IN A FERMENTATION METHOD

SOUCHE BACTÉRIENNE POUR LA LIBÉRATION D'UNE PROTÉINE RECOMBINANTE DANS UN PROCÉDÉ DE FERMENTATION

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(43) Veröffentlichungstag der Anmeldung:
**15.06.2022 Patentblatt 2022/24**

(73) Patentinhaber: **Wacker Chemie AG**
**81671 München (DE)**

(72) Erfinder:
• **KOCH, Johanna**
**81377 München (DE)**
• **KUJAU, Marian**
**07745 Jena (DE)**

(74) Vertreter: **Belz, Ferdinand et al**
**Wacker Chemie AG**
**Gisela-Stein-Straße 1**
**81671 München (DE)**

(56) Entgegenhaltungen:
**EP-A1- 2 204 441   WO-A1-2013/007388**

• **SINGH S K ET AL: "Three redundant murein endopeptidases catalyse an essential cleavage step in peptidoglycan synthesis of Escherichia coli K12 : Essential murein endopeptidases of E.?coli", MOLECULAR MICROBIOLOGY., vol. 86, no. 5, 4 November 2012 (2012-11-04), GB, pages 1036 - 1051, XP055677639, ISSN: 0950-382X, DOI: 10.1111/mmi.12058**
• **YANG H ET AL: "Improving extracellular protein production inEscherichia coliby overexpressing D,D-carboxypeptidase to perturb peptidoglycan network synthesis and structure", APPLIED MICROBIOLOGY AND BIOTECHNOLOGY, SPRINGER BERLIN HEIDELBERG, BERLIN/ HEIDELBERG, vol. 103, no. 2, 12 November 2018 (2018-11-12), pages 793 - 806, XP036692548, ISSN: 0175-7598, [retrieved on 20181112], DOI: 10.1007/S00253-018-9510-7**
• **OJIMA Y ET AL: "Quantitative Evaluation of Recombinant Protein Packaged into Outer Membrane Vesicles of Escherichia coli Cells", BIOTECHNOLOGY PROGRESS, vol. 34, no. 1, 21 August 2017 (2017-08-21), pages 51 - 57, XP055677649, ISSN: 8756-7938, DOI: 10.1002/ btpr.2536**

EP 4 010 500 B1

• KLEINER-GROTE G R M ET AL: "Secretion of recombinant proteins from E. coli", ENGINEERING IN LIFE SCIENCES, vol. 18, no. 8, 14 April 2018 (2018-04-14), DE, pages 532 - 550, XP055677654, ISSN: 1618-0240, DOI: 10.1002/ elsc.201700200
• KARYOLAIMOS A ET AL: "Enhancing Recombinant Protein Yields in the E. coli Periplasm by Combining Signal Peptide and Production Rate Screening", FRONTIERS IN MICROBIOLOGY, vol. 10, 23 July 2019 (2019-07-23), XP055677653, ISSN: 1664-302X, DOI: 10.3389/fmicb.2019.01511

Bemerkungen:
 Das gesamte Dokument mit Referenztabelle(n) und Sequenzliste(n) kann von der Webseite des EPA heruntergeladen werden

Bemerkungen:
 Das gesamte Dokument mit Referenztabelle(n) und Sequenzliste(n) kann von der Webseite des EPA heruntergeladen werden

**Beschreibung**

Die Erfindung betrifft ein Verfahren zur fermentativen Produktion von rekombinanten Proteinen wie im beigefügten Anspruchssatz beschrieben .

Rekombinante Proteine können in Bakterien, wegen ihrer kurzen Generationszeit und der einfachen Handhabung, im Vergleich zu Säugerzellkulturen kostengünstig hergestellt werden. Aufgrund seiner sehr gut untersuchten Genetik und Physiologie ist das gramnegative Enterobakterium *Escherichia coli* gegenwärtig der am häufigsten verwendete Organismus zur Produktion rekombinanter Proteine. Besonders attraktiv sind Produktionsverfahren für rekombinante Proteine in *E. coli,* bei denen das Zielprotein in hoher Ausbeute und in der korrekten Faltung direkt ins Fermentationsmedium freigesetzt wird, da hier aufwändige Zellaufschluss- und Proteinfaltungsprozesse vermieden werden.

In der Literatur sind eine Reihe von *E. coli*-Stämmen offenbart, durch die eine Freisetzung von rekombinanten Proteinen ins Kulturmedium erreicht wird. Es handelt sich dabei um Mutanten von *E. coli,* die einen permanenten Defekt in der äußeren Membran aufweisen und deshalb periplasmatische Proteine teilweise in das Kulturmedium entlassen. Es handelt sich dabei um einen unspezifischen Mechanismus. Ein Beispiel für solche "leaky"-Mutanten sind Stämme mit Defekten im Tol-Pal-Komplex, z.B. tol- und pal-Deletions-Mutanten. Es ist bekannt, dass tol- und pal-Deletions-Mutanten zelleigene periplasmatische Proteine, in das Kulturmedium entlassen. Solche Stämme sind extrem empfindlich gegenüber EDTA, verschiedenen Detergenzien und Antibiotika und weisen Wachstumsdefekte auf (Clavel et al. 1998, Mol. Microbiol. 29, S. 359-367; Chen et al. 2014, Microb. Biotechnol. 7, S. 360-370). Daher sind sie zur Kultivierung bei hohen Zelldichten und über einen längeren Zeitraum nur eingeschränkt geeignet.

Aus diesem Grund ist es bevorzugt, Stämme zu verwenden, die Proteine in das Kulturmedium freisetzen können, ohne permanente Defekte aufzuweisen. Die zu einem bestimmten Zeitpunkt in einem Produktionsprozess induzierbare Freisetzung von rekombinanten Proteinen kann erreicht werden, indem die Peptidoglykanschicht, die ein wesentlicher Stabilitätsfaktor der bakteriellen Zellhülle ist, geschwächt wird.

Die Peptidoglykan- oder Murein-Schicht, ist ein vernetztes Makromolekül, das in der Zellhülle von Gram-positiven und Gram-negativen Bakterien vorkommt und dieser Festigkeit verleiht. Das Peptidoglykan besteht aus Strängen aus miteinander verknüpften Molekülen von N-Acetylglukosamin und N-Acetylmuraminsäure, die als lineare Kette das Rückgrat bilden. Die N-Acetylmuraminsäure-Reste benachbarter Stränge werden über ein Oligopeptid miteinander verknüpft (Silhavy et al. 2010, Cold Spring Harb. Perspect. Biol. 2(5)).

Bei Peptidoglykan-Hydrolasen handelt es sich um eine Gruppe von Enzymen, die kovalente Bindungen der Peptidoglykanschicht spalten können (Vollmer, FEMS Microbiol Rev. 32, 2008, S. 259-286). Je nachdem welche Art von Bindung gespalten wird, werden die Peptidoglykan-Hydrolasen in verschiedene Klassen unterteilt. Glykosidasen (N-Acetylglukosaminidasen, lytische Transglykosylasen), zu denen auch die Lysozyme gehören, spalten die glykosidischen Bindungen des Peptidoglykan-Rückgrats, das aus N-Acetylmuraminsäure und N-Acetylglukosamin-Einheiten aufgebaut ist. Amidasen (N-Acetylmuramyl-L-Alanin-Amidasen) spalten die Amid-Bindung zwischen der N-Acetylmuraminsäure des Peptidoglykan-Rückgrats und dem L-Alanin der Peptid-Querverbindung. Peptidasen (Carboxy- und Endopeptidasen) dagegen spalten die Peptidbindungen innerhalb der Peptid-Querverbindung.

Ein bekanntes Beispiel für den Einsatz einer Peptidoglykan-Hydrolase im Stand der Technik, ist die Zugabe von Lysozym zu Zellen nach der Ernte einer Bakterienkultur, um diese aufzuschließen (Pierce et al 1997, J Biotechnol 58, S. 1-11).

So wird in der Patentschrift CN 104762285 B ClyN, eine Fusion aus Lysozym- und Peptidoglykan-Bindedomäne, induzierbar produziert und auf diese Weise rekombinant hergestelltes, cytoplasmatisches grün fluoreszierendes Protein (GFP) aus den Zellen in das Kulturmedium freigesetzt.

Ein weiteres bekanntes Prinzip ist die Co-Transformation von *E. coli* mit einem Vektor zur Produktion eines rekombinanten Proteins und einem Vektor zur Produktion von Lysozym aus dem T4 Phagen zur Freisetzung des rekombinanten Proteins wie beispielsweise von cytoplasmatischer β-Glucuronidase (Morita et al 2001, Biotechnol. Prog. 17, S. 573-576) oder eines anti-CD18-Antikörper Fragments bzw. IGF-I (EP 1 124 941).

Die Produktion von Lysozym führt zur Lyse der Zellen, daher haben diese Ansätze den großen Nachteil, dass die Freisetzung der rekombinanten Proteine begleitet wird von der Freisetzung anderer intrazellulärer Bestandteile, wie Wirtszellproteinen und DNA. Hierdurch wird der Kulturüberstand verunreinigt, was Schwierigkeiten in der Aufarbeitung, insbesondere durch eine erhöhte Viskosität nach sich zieht.

Dieser nachteilige Effekt wird in CN 104762285 B für die Freisetzung von GFP durch ClyN beschrieben, bei der die Lebendzellzahl nach Induktion der ClyN-Produktion auf 0,2 % gegenüber einer Kultur ohne Induktion der ClyN-Produktion zurückgeht. Dabei sinkt die optische Dichte der *E. coli*-Kultur exprimierend ClyN aufgrund der Zelllyse innerhalb von 30 min um ca. zwei Drittel des Werts vor Beginn der ClyN-Produktion. Nach 5 h beträgt die optische Dichte der Kultur exprimierend ClyN nur ca. 20 % der optischen Dichte einer Vergleichskultur ohne ClyN-Produktion. Die Freisetzung von β-Glucuronidase durch T4-Phagen-Lysozym in Morita et al geht einher mit einer signifikant geringeren optischen Dichte der Kultur.

Der gleiche nachteilige Effekt wird in EP 1 124 941 für die Freisetzung des anti-CD18-Antikörper Fragments berichtet, wo die Überproduktion des T4-Phagen-Lysozyms zu einem Rückgang der Zelldichte auf 30 % des maximal-Werts führte. Bei der Freisetzung von IGF-I durch T4-Phagen-Lysozyms wurde ebenfalls ein starker Rückgang der Zelldichte durch Lyse beobachtet, und bei der mikroskopischen Analyse nur noch wenige intakte Zellen gefunden.

Peptidoglykan-Peptidasen haben Auswirkungen auf das Zellwachstum und die Lebensfähigkeit von Bakterienzellen (Singh et al., 2012, Mol. Microbiol., 86, S. 1036-1051). WO2013/007388 beschreibt einen E. coli-Stamm zur Produktion von Antikörpern, der eine nicht-functionelle Spr-Mutante exprimiert. Weiter sind E. coli-Stämme bekannt, welche die Peptidoglykan-spaltenden D,D-Carboxypeptidasen DacA oder DacB zusammen mit einem rekombinanten Protein ohne Signalsequenz exprimieren (Yang et al., 2018, Appl. Microbiol. Biotech, 103, S. 793-806). Ebenfalls bekannt ist ein E. coli-Stamm, der ein Fusionsprotein bestehend aus einem Membranprotein und GPF sowie zusätzlich eine Spr Peptidoglykan-Peptidase exprimiert (Ojima et al., 2017, Biotech. Progr., 34, S51-57). Schließlich sind eine Reihe von weiteren Proteinen bekannt, die zu einer Permeabilisierung und Destabilisierung der Bakterienzellhülle sowie zu einer verstärkten Freisetzung rekombinant exprimierter Proteine führen (Kleiner-Grote et al., 2018, Eng. Life Scie.,18, S. 532-550).

Aufgabe der vorliegenden Erfindung ist es, ein Verfahren bereitzustellen, in dem ein rekombinantes Protein verglichen mit einer Wildtyp-Wirtszelle in erhöhter Ausbeute in das Kulturmedium freigesetzt wird, ohne dass es zu einer starken Zelllyse der Wirtszellen kommt.

Diese Aufgabe wird gelöst durch ein Verfahren zur fermentativen Produktion von rekombinanten Proteinen wie im beigefügten Anspruchssatz beschrieben

Der Bakterienstamm ist dadurch gekennzeichnet, dass es sich bei dem Bakterienstamm um einen Stamm der Art *Escherichia* coli handelt.

Als Wildtyp-Gen wird die Form des Gens bezeichnet, die natürlicherweise durch die Evolution entstanden und im Wildtyp-Bakteriengenom vorhanden ist.

Vom Gen wird das Vorläufer-Protein exprimiert, das die Aminosäuresequenz des gesamten Proteins inklusive der Aminosäuresequenz für die Signalsequenz umfasst und keine posttranslationalen Modifikationen trägt.

Der Begriff reifes Protein bezeichnet das Protein, das die Aminosäuresequenz des gesamten Proteins, dem die Signalsequenz entfernt wurde, umfasst und bei dem eventuelle posttranslationale Modifikationen vorhanden sind. Eine solche posttranslationale Modifikation ist beispielsweise die Modifizierung mit einem Membrananker.

Im Rahmen dieser Erfindung bezeichnet der Begriff Volllänge-Protein das reife Protein.

Erfindungsgemäß codiert der im Bakterienstamm enthaltene zusätzliche offene Leserahmen ein Signalpeptid und eine Peptidoglykan-Peptidase, die zu den Peptidoglykan-Hydrolasen zählt. Im Folgenden ist mit dem Begriff "Peptidoglykan-Peptidase" immer das von diesem zusätzlichen ORF exprimierte zusätzliche Protein in seiner reifen Form, d.h. nach Abspalten des Signalpeptids gemeint, nicht das Protein, das durch das im bakteriellen Wildtyp-Genom vorhandene und exprimierte Gen codiert wird. Beispiele für Gene codierend Peptidoglykan-Peptidasen in *E. coli* sind spr, ydhO, yebA, dacA, dacC, dacD, yfeW, pbpG, mepA, dacB, ampH und ldcA.

1. In einer bevorzugten Ausführungsform handelt es sich bei der vom zusätzlichen ORF codierten Peptidoglykan-Peptidase um ein Wildtyp-Volllänge-Protein, das die Bindung zwischen dem D-Alanin und der meso-Diaminopimelinsäure der Peptidseitenkette spaltet, wie Spr (Uniprot-Eintrag P0AFV4) oder YdhO (Uniprot-Eintrag P76190) mit der aus den angegebenen Datenbankeinträgen bekannten Aminosäuresequenz,

2. in einer alternativ bevorzugten Ausführungsform handelt es sich bei der vom zusätzlichen ORF codierten Peptidoglykan-Peptidase um eine mutierte Form eines Wildtyp-Volllänge-Proteins mit einer Sequenzidentität von mindestens 30%, bevorzugt mindestens 70% zur Aminosäuresequenz des entsprechenden Wildtyp-Volllänge-Proteins,

3. in einer weiteren bevorzugten Ausführungsform handelt es sich bei der vom zusätzlichen ORF codierten Peptidoglykan-Peptidase um ein Fragment eines Wildtyp-Volllänge-Proteins,

4. in einer darüber hinaus bevorzugten Ausführungsform handelt es sich bei der vom zusätzlichen ORF codierten Peptidoglykan-Peptidase um eine mutierte Version eines Fragments eines Wildtyp-Proteins mit einer Sequenzidentität von mindestens 30%, bevorzugt mindestens 70% zur Aminosäuresequenz des entsprechenden Fragments eines Wildtyp-Volllänge-Proteins,

wobei in allen Fällen 1-4 das vom zusätzlichen ORF codierte und als Peptidoglykan-Peptidase gewählte Protein eine Peptidoglykan-Peptidase-Aktivität aufweist. Methoden wie diese Aktivität nachgewiesen werden kann sind dem Fachmann aus der Literatur bekannt (Gonzalez-Leiza et al 2011, J Bacteriol 193, S. 6887-94).

Bei den Mutationen auf Aminosäureebene, wie sie z.B. unter Punkt 2 und Punkt 4 beschrieben sind, kann es sich um Substitutionen, Deletionen und/oder Insertionen im Vergleich zum Wildtyp-Peptidoglykan-Peptidase-Volllänge-Protein oder Fragmenten hiervon handeln.

Beispiele für Peptidoglykan-Peptidasen sind Spr und YdhO, die die Bindung zwischen D-Alanin und Meso-Diaminopimelinsäure in den Peptid-Querverbindungen der bakteriellen PeptidoglykanSchicht spalten (Singh et al. 2012, Mol. Microbiol. 86, S. 1036-1051) und so den Einbau neuer Stränge in das bestehende Peptidoglykan-Netzwerk und damit Zellwachstum ermöglichen.

Das spr-Gen aus *E. coli* codiert für ein Spr-Protein mit 188 Aminosäuren (Uniprot P0AFV4), wobei die Aminosäuren 1-26 die Signalsequenz für den Transport aus dem Cytosol in das Periplasma bilden. Im reifen Spr-Protein wird die Signalsequenz proteolytisch abgespalten und zusätzlich der Cysteinrest an Position 1 des reifen Proteins (Position 27 der Gesamtsequenz) mit einem Membrananker modifiziert, über den das Spr-Protein in der äußeren Membran verankert wird. Das reife Spr-Protein besteht dementsprechend aus 162 Aminosäuren und wird im Rahmen dieser Erfindung als Spr-Volllänge-Protein bezeichnet.

Das *ydhO*-Gen codiert für ein YdhO-Protein mit 271 Aminosäuren (Uniprot P76190). Die Aminosäuren 1-27 sind als Periplasma-Export-Signalpeptid vorhergesagt. Das reife YdhO-Protein besteht dementsprechend aus 244 Aminosäuren und wird im Rahmen dieser Erfindung als YdhO-Volllänge-Protein bezeichnet.

Die das Spr-Vorläufer-Protein codierende DNS-Sequenz ist in SEQ ID Nr. 4 angegeben, die das YdhO-Vorläufer-Protein codierende DNS-Sequenz in SEQ ID Nr. 6. Die Aminosäuresequenz des Spr-Vorläufer-Proteins ist in SEQ ID Nr. 5 angegeben, wobei die Aminosäuren 1-26 die Signalsequenz bilden und die Aminosäuren 27-188 das reife Spr-Protein (gleichzusetzen mit dem Spr-Volllänge-Protein). Die Aminosäuresequenz des YdhO-Vorläufer-Proteins ist in SEQ ID Nr. 7 angegeben, wobei die Aminosäuren 1-27 die Signalsequenz bilden und die Aminosäuren 28-271 das reife YdhO-Protein (gleichzusetzen mit dem YdhO-Volllänge-Protein).

Es handelt sich bei der Peptidoglykan-Peptidase um (i) Spr, (ii) YdhO oder (iii) Spr und YdhO. Dabei handelt es sich bei Spr bzw. YdhO um das jeweils in der Datenbanksequenz angegebene Wildtyp-Volllänge-Protein, eine mutierte Form des Wildtyp-Volllänge-Proteins mit einer Sequenzidentität von mindestens 30%, bevorzugt mindestens 70% zur Aminosäuresequenz des Wildtyp-Volllänge-Proteins, ein Fragment des Wildtyp-Volllänge-Proteins oder eine mutierte Version eines Fragments des Wildtyp-Volllänge-Proteins mit einer Sequenzidentität von mindestens 30%, bevorzugt mindestens 70% zur Aminosäuresequenz eines Fragments des Wildtyp-Volllänge-Proteins, wobei das gewählte Protein Murein-DD-Endopeptidase-Aktivität aufweist. Insbesondere bevorzugt handelt es sich bei Spr um die in SEQ ID Nr. 5 von Aminosäuren 27-188 angegebene Sequenz und bei YdhO um die in SEQ ID Nr. 7 von Aminosäuren 28-271 angegebene Sequenz.

In der bevorzugten Ausführungsform, in der es sich bei der Peptidoglykan-Peptidase um Spr handelt, ist es besonders bevorzugt, dass das Spr-Protein mit der äußeren Membran interagiert.

Die Begriffe DNS und DNA werden im Rahmen dieser Erfindung synonym verwendet.

Bei den DNA-Sequenzen codierend die erfindungsgemäße Peptidoglykan-Peptidase handelt es sich bevorzugt um eine DNA-Sequenz, die aus *Escherichia coli* stammt.

Als offener Leserahmen (*open reading frame,* ORF) wird derjenige Bereich der DNS bzw. RNS bezeichnet, der zwischen einem Startcodon und einem Stoppcodon liegt und für die Aminosäuresequenz eines Proteins codiert. Der ORF wird auch als codierende Region bezeichnet.

ORFs werden von nicht-codierenden Bereichen umgeben. Als Gen wird der DNS-Abschnitt bezeichnet, der alle Grundinformationen zur Herstellung einer biologisch aktiven RNS enthält. Ein Gen enthält den DNS-Abschnitt, von dem durch Transkription eine einzelsträngige RNS-Kopie hergestellt wird und die Expressionssignale, die an der Regulation dieses Kopiervorgangs beteiligt sind. Zu den Expressionssignalen zählen z.B. mindestens ein Promotor, ein Transkriptions-, ein Translationsstart und eine Ribosomenbindestelle. Des Weiteren sind als Expressionssignale ein Terminator und ein oder mehrere Operatoren möglich.

Für einen funktionellen Promotor gilt, dass der unter der Regulation dieses Promotors stehende ORF in eine RNS transkribiert wird.

Als monocistronisch wird eine Boten-RNS (mRNA) bezeichnet, die nur einen offenen Leserahmen enthält.

Ein Operon ist eine Funktionseinheit der DNS, die mehrere ORFs, einen Promotor und gegebenenfalls weitere Expressionssignale umfasst.

Der Bakterienstamm kann (1) einen ORF codierend für ein Signalpeptid und ein rekombinantes Protein, (2) mehrere ORFs codierend für dasselbe Signalpeptid und rekombinante Protein oder (3) mehrere ORFs codierend für jeweils ein unterschiedliches Signalpeptid und rekombinantes Protein enthalten. Während die 2. Möglichkeit z.B. zur Erhöhung der Expression und Ausbeute des rekombinanten Proteins verwendet wird, findet die 3. Möglichkeit besonders bei der Expression von Proteinen, die aus mehreren Polypeptiden (Untereinheiten) aufgebaut sind, Anwendung. Ein Beispiel ist die Expression von Antikörperfragmenten, die dann nach dem Transport aus dem Cytoplasma assembliert werden.

Jeder dieser ORFs kann in einem separaten Gen liegen, es können auch mehrere ORFs in einem Operon organisiert sein, d.h. von gemeinsamen Expressionssignalen reguliert werden.

Im Fall, dass das rekombinante Protein aus mehreren Polypeptidketten (Untereinheiten) aufgebaut ist, ist es bevorzugt, dass die ORFs der einzelnen Peptidketten (Untereinheiten), jeweils verknüpft mit einem Signalpeptid, in einem Operon organisiert sind.

Jedes Operon kann zudem einen ORF codierend ein Signalpeptid und eine Peptidoglykan-Peptidase enthalten.

Es ist bevorzugt, dass der ORF codierend ein Signalpeptid und die Peptidoglykan-Peptidase als monocistronische Boten-RNS transkribiert wird. D. h., dass die Peptidoglykan-Peptidase von einem separaten, eigenen Gen exprimiert wird. In der bevorzugten Ausführungsform, in der es sich bei der Peptidoglykan-Peptidase um Spr oder YdhO handelt, wird das entsprechende Gen als spr-Gen oder *ydhO*-Gen bezeichnet.

Der oder die ORFs codierend das oder die Signalpeptid(e) und rekombinante(n) Protein(e) und das oder die Signalpeptid(e) und Peptidoglykan-Peptidase(n) können chromosomal oder von einem Plasmid exprimiert werden. Wenn es sich um getrennte Gene handelt, können diese auch von getrennten, in Bezug auf Replikationsursprung und Selektionsmarker miteinander kompatiblen, Plasmiden exprimiert werden. Bevorzugt sind die ORFs Plasmid-codiert.

Der Ursprung der DNS-Sequenz des erfindungsgemäßen ORFs codierend eine Peptidoglykan-Peptidase ist nicht beschränkt auf den zur Produktion des rekombinanten Proteins verwendeten Bakterienstamm, solange die entsprechende Peptidoglykan-Peptidase in dem zur Produktion des rekombinanten Proteins verwendeten Bakterienstamm funktionell ist, d.h. Murein-DD-Endopeptidase-Aktivität aufweist (s.o.). Bevorzugt handelt es sich bei der Sequenz des ORFs codierend das Peptidoglykan-Peptidase-Protein um eine DNS-Sequenz, die aus dem gleichen Organismus, der auch zur Produktion der rekombinanten Proteine verwendet wird, stammt.

Der im Rahmen dieser Erfindung in der Einzahl verwendete Begriff "ein/der ORF codierend für ein Signalpeptid und ein rekombinantes Protein" und "ein/das rekombinante/s Protein" kann auch mehrere ORFs und/oder mehrere verschiedene rekombinante Proteine, die jeweils von einem Signalpeptid angeführt werden, bedeuten. Bevorzugt handelt es sich um 1 bis 3 verschiedene rekombinante Proteine, besonders bevorzugt um 1 rekombinantes Protein oder 2 verschiedene rekombinante Proteine. Die Klonierung und Expression von rekombinanten Proteinen in Bakterien erfolgt wie im Stand der Technik beschrieben. Das rekombinante Protein besitzt beispielsweise eine Signalsequenz für den Transport in das Periplasma.

Beim rekombinanten Protein handelt es sich um ein Protein, das vom Wildtyp-Bakteriengenom natürlicherweise entweder gar nicht oder in anderer Menge exprimiert wird. Das Bakterium dient der Herstellung des rekombinanten Proteins, wobei dieses erfindungsgemäß ins Kulturmedium freigesetzt wird.

Das erfindungsgemäße Fermentationsverfahren hat den Vorteil, dass der verwendete Bakterienstamm zusammen mit dem rekombinanten Protein eine Peptidoglykan-Peptidase als zusätzliches Protein exprimiert. Der Begriff "zusätzlich" bezieht sich darauf, dass im Bakterienstamm ein weiterer bzw. "zusätzlicher" offener Leserahmen codierend für eine Peptidoglykan-Peptidase zusätzlich zum Wildtyp-Gen vorhanden ist. Bevorzugt liegt dieser zusätzliche offene Leserahmen codierend für eine Peptidoglykan-Peptidase auf einem Plasmid.

Durch ihre Signalsequenz ist die Peptidoglykan-Peptidase im Periplasma lokalisiert und führt durch die Spaltung der Peptid-Querverbindungen zu einer Destabilisierung der Peptidoglykanschicht. Trotz der Destabilisierung der Zellhülle des Bakteriums wurde keine erhöhte Zelllyse beobachtet und in den Experimenten blieb das Zelltrockengewicht des Bakterienstammes exprimierend ein rekombinantes Protein und die Peptidoglykan-Peptidase im Vergleich zum Bakterienstamm, exprimierend nur das rekombinante Protein, gleich. Durch die Destabilisierung der Zellhülle des Bakteriums können rekombinante Proteine aus dem Periplasma der Zelle freigesetzt werden und lassen sich in erhöhter Ausbeute aus dem Kulturüberstand isolieren. Auf diese Weise können gegenüber dem Stand der Technik erhöhte Produktausbeuten im Kulturüberstand erzielt werden, ohne dass es zu einem beträchtlichen Absterben der Bakterienzellen kommt. Unter erhöhter Ausbeute ist zu verstehen, dass mit dem erfindungsgemäßen Bakterienstamm mindestens 110%, besonders bevorzugt mindestens 150% und insbesondere bevorzugt mindestens 200% der Menge an rekombinantem Protein in das Kulturmedium freigesetzt wird, verglichen damit, was nach dem gegenwärtigen Stand der Technik mit einem Wildtyp-Bakterienstamm enthaltend das gleiche Gen für ein rekombinantes Protein produziert werden kann. Das heißt, die Ausbeute an rekombinantem Protein, das in das Kulturmedium freigesetzt wird, ist mindestens 1,1-mal, besonders bevorzugt mindestens 1,5-mal und insbesondere bevorzugt mindestens 2-mal so hoch wie die Ausbeute, die mit entsprechenden Bakterienstämmen ohne Überproduktion einer Peptidoglykan-Peptidase erreicht werden kann.

In der vorliegenden Erfindung wurde CGTase als ein Beispiel für ein rekombinantes Protein verwendet. Der Vergleich der CGTase-Ausbeute in Beispiel 1 zeigt, dass die Ausbeute an CGTase gemessen in U/ml bei Verwendung des W3110/pCGT-Spr-Bakterienstamms im Vergleich zum W3110/pCGT-Bakterienstamm um knapp das 10-fache erhöht ist (s. Tab. 1). Weder die Zelltrockenmasse (CDW), noch die Lebendzellzahl wurde durch die zusätzliche Produktion des Spr-Proteins gegenüber dem Kontrollstamm gesenkt. Der Stamm W3110/pCGT-YdhO erzielte nahezu die gleiche CGTase-Ausbeute wie der Stamm W3110/pCGT-Spr (s. Tab. 2), ohne die Zelltrockenmasse gegenüber dem Kontrollansatz mit dem W3110/pCGT-Bakterienstamm wesentlich zu beeinflussen.

Beispiel 2 bestätigt auch für die Produktion eines Fab-Antikörperfragments als rekombinantes Protein, in diesem Fall des CD154-Fab-Fragments, dass mit dem die Peptidoglykan-Peptidase Spr exprimierenden Stamm W3110/pJF118ut-CD154-Spr eine wesentlich höhere Ausbeute an rekombinantem Protein im Vergleich zum Stamm W3110/pJF118ut-CD154, in diesem Fall gut 200-fach, erzielt werden konnte (Tab. 3). Gleichzeitig sank die Zelltrockenmasse nur wenig (Tab. 4).

$OD_{600}$ bezeichnet die optische Dichte der Zellkultur bestimmt mit Hilfe eines Spektrophotometers bei 600 nm und ist ein Maß für die Menge an Zellen je Volumeneinheit (Zellkonzentration, Zelldichte), die wiederum ein Maß für die Zellteilungsaktivität sowie ein Maß für die Zelllyse ist, wobei Zelllyse zu einer Abnahme der Zelldichte führt.

CN 104762285 B zeigt dagegen in Fig. 2, dass der OD600-Wert, also die Zelldichte, nach Induktion der Expression von ClyN wesentlich absinkt, was auf eine starke Zelllyse schließen lässt.

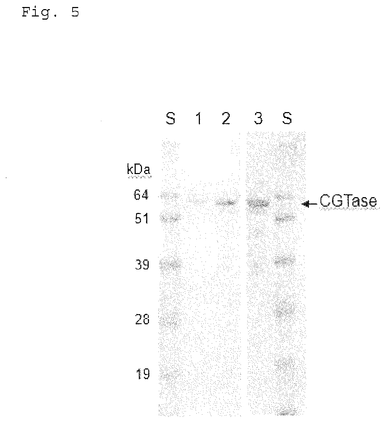

Da eine starke Zelllyse zur Freisetzung von Wirtszellproteinen führt, kommt es zu einer Verunreinigung des rekombinanten Proteins mit Wirtszellproteinen. Außerdem führt die Freisetzung von DNA zu einem Anstieg der Viskosität im Kulturmedium, was die weitere Aufarbeitung erschwert. Da der erfindungsgemäße Bakterienstamm keine erhöhte Zelllyserate zeigt, kommt es bei Verwendung dieses Stamms zur Produktion rekombinanter Proteine nicht zu einer Verunreinigung des rekombinanten Proteins mit Wirtszellproteinen. Dies bestätigt die Analyse des Proteingehalts des Kulturüberstands mittels SDS-PAGE in Beispiel 1 (Fig. 5), die zeigt, dass bei Verwendung des erfindungsgemäßen Bakterienstamms keine starke Verunreinigung des rekombinanten Proteins durch Wirtszellproteine auftrat. Auch die zusätzlich exprimierte Peptidoglykan-Peptidase wurde nicht verstärkt freigesetzt.

Um die Freisetzung des rekombinanten Proteins in das Kulturmedium zu erzielen, ist es notwendig, dass sowohl das rekombinante Protein, als auch die Vorläufer-Peptidoglykan-Peptidase nach der Proteinbiosynthese im Cytosol in das Periplasma transportiert werden. Für den Transport in das Periplasma ist es notwendig, das 5'-Ende der codierenden DNS-Sequenz des rekombinanten Proteins und das 5'-Ende der codierenden DNS-Sequenz der Peptidoglykan-Peptidase *in frame* mit dem 3'-Ende einer Signalsequenz für den Protein-Export zu verknüpfen. Dazu sind prinzipiell alle Signalsequenzen geeignet, die in dem verwendeten Bakterienstamm eine Translokation des Zielproteins mit Hilfe des Sec-, oder Tat-Apparats ermöglichen. Verschiedene Signalsequenzen sind im Stand der Technik beschrieben, so z.B. die Signalsequenzen der folgenden Gene: phoA, ompA, pelB, ompF, ompT, lamB, malE, Staphylococcal protein A, StII und andere (Choi und Lee 2004, Appl. Microbiol. Biotechnol. 64, S. 625-635). Erfindungsgemäß bevorzugt für rekombinante Proteine ist die Signalsequenz des phoA- oder des ompA-Gens von *E. coli* oder die Signalsequenz für eine Cyclodextrin-Glycosyltransferase ($\alpha$-CGTase) aus *Klebsiella pneumoniae* M5a1 bzw. davon abgeleitete Signalsequenzen, die in US 2008/0076157 als SEQ ID Nr. 1 und 3 offenbart sind. Bevorzugt trägt die Peptidoglykan-Peptidase in ihrer Vorläufer-Form die für das jeweilige Protein native Signalsequenz. Wenn es sich bei der Peptidoglykan-Peptidase um Spr bzw. YdhO handelt, handelt es sich bei der Vorläufer-Form der jeweiligen Peptidoglykan-Peptidase um die in SEQ ID Nr. 5 bzw. 7. angegebene Sequenz. Bevorzugt tragen das rekombinante Protein und die Peptidoglykan-Peptidase eine unterschiedliche Signalsequenz, wobei beide Signalsequenzen den Proteinexport bewirken.

Die Expression der ORFs codierend die Peptidoglykan-Peptidase und das rekombinante Protein können von einem, von zwei gleichen oder zwei verschiedenen Promotoren kontrolliert werden. Es ist bevorzugt, dass die ORFs für die Peptidoglykan-Peptidase und für das rekombinante Protein unter Kontrolle von zwei verschiedenen Promotoren stehen.

Der DNS-Abschnitt codierend die Peptidoglykan-Peptidase kann zunächst mittels PCR unter Verwendung von Oligonukleotiden als Primer und einer DNS-Matrize, codierend die Peptidoglykan-Peptidase, beispielsweise genomische DNA isoliert aus *E. coli,* amplifiziert und anschließend mit gängigen molekularbiologischen Techniken jeweils mit dem DNS-Molekül, das die Sequenz eines Signalpeptids umfasst und das auf analoge Weise erzeugt wurde, derart verknüpft werden, dass eine in frame-Fusion entsteht. Alternativ kann auch das gesamte DNS-Molekül mittels Gensynthese hergestellt werden. Dieses DNS-Molekül bestehend aus der jeweiligen Signalsequenz und der codierenden Sequenz für das reife Protein kann dann entweder in einen Vektor, z.B. ein Plasmid, eingebracht werden oder direkt nach bekannten Methoden in das Chromosom des Bakterienstamms integriert werden. Bevorzugt wird das DNS-Molekül in ein Plasmid eingebracht, wie etwa einem Abkömmling von bekannten Expressionsvektoren, wie pJF118EH, pKK223-3, pUC18, pBR322, pACYC184, pASK-IBA3 oder pET. Plasmide werden unter Anwendung von dem Fachmann bekannten Methoden in die Bakterienzellen eingebracht (Transformation).

Die eingesetzten Plasmide können Selektionsmarker tragen. Als Selektionsmarker geeignet sind Gene, die für eine Resistenz gegenüber Antibiotika wie z.B. Ampicillin, Tetracyclin, Chloramphenicol, Kanamycin oder andere codieren. Bevorzugt enthält das Plasmid ein Gen, dessen Expression Tetracyclin-Resistenz vermittelt. Weiterhin sind Auxotrophie-Marker als Selektionsmarker geeignet, die für ein essentielles Gen codieren, das im jeweiligen Bakterienstamm, der das Plasmid enthält, deletiert ist. Werden zwei Plasmide in die Zellen transformiert, wird bevorzugt mit zwei verschiedenen Antibiotikaresistenzen, einer Antibiotikaresistenz und einem Auxotrophie-Marker oder zwei verschiedenen Auxotrophie-Markern auf das Vorhandensein beider Plasmide selektiert.

Als Promotoren eignen sich alle dem Fachmann bekannten Promotoren, wie konstitutive Promotoren, wie z.B. der GAPDH-Promotor, oder induzierbare Promotoren wie z.B. der lac-, tac-, trc-, lambda PL, ara-, cumate- oder tet-Promotor oder davon abgeleitete Sequenzen. Die ORFs codierend das rekombinante Protein und der ORF codierend die Peptidoglykan-Peptidase können als Operon von einem Promotor kontrolliert werden oder unter Kontrolle von verschiedenen Promotoren stehen. Bevorzugt handelt es sich beim funktionellen Promotor, der die Expression des offenen Leserahmens codierend die Peptidoglykan-Peptidase kontrolliert, um einen induzierbaren Promotor.

Bevorzugt werden die ORFs codierend das rekombinante Protein und der ORF codierend die Peptidoglykan-Peptidase durch verschiedene induzierbare Promotoren kontrolliert. Besonders bevorzugt wird das rekombinante Protein unter Kontrolle des tac-Promotors und die Peptidoglykan-Peptidase unter Kontrolle des ara (Arabinose)-Promotors exprimiert.

Bevorzugt handelt es sich beim rekombinanten Protein um ein heterologes Protein. Unter heterologen Proteinen sind Proteine zu verstehen, die nicht zum Proteom, d.h. der gesamten natürlichen Protein-Ausstattung des Bakterienstamms, bevorzugt eines *E. coli* K12-Stamms, gehören. Alle natürlicherweise in dem verwendeten Bakterienstamm, z.B. im *E. coli* K12-Stamm, vorkommenden Proteine lassen sich aus den bekannten Genomsequenzen ableiten (z.B. aus dem Genbank-Eintrag unter der Accession No. NC_000913 für *E. coli* K12).

Als heterologe Proteine sind eukaryontische Proteine, die eine oder mehrere Disulfid-Brücken enthalten, besonders bevorzugt. Insbesondere bevorzugt sind eukaryontische Proteine, die in ihrer funktionellen Form als Di- oder Multimere vorliegen.

Zu den wichtigsten heterologen Proteinklassen zählen Antikörper und deren Fragmente, Cytokine, Wachstumsfaktoren, Proteinkinasen, Proteinhormone, Lipokaline, Antikaline, Enzyme, Bindeproteine und molekulare Scaffolds und davon abgeleitete Proteine. Beispiele für diese Proteinklassen sind u.a. heavy-chain Antikörper und deren Fragmente (z.B. Nano-bodies), einzelkettige Antikörper, Interferone, Interleukine, Interleukinrezeptoren, Interleukinrezeptor Antagonisten, G-CSF, GM-CSF, M-CSF, Leukämieinhibitoren, Stammzellenwachstumsfaktoren, Tumornekrosefaktoren, Wachstumshormone, insulinartige Wachstumsfaktoren, Fibroblastenwachstumsfaktoren, von Blutplättchen abstammende Wachstumsfaktoren, transformierende Wachstumsfaktoren, Hepatocyten-Wachstumsfaktoren, knochenmorphogenetische Faktoren, Nervenwachstumsfaktoren, vom Gehirn abstammende neurotrophe Faktoren (BDNF), von Gliazelllinien abstammende neurotrophe Faktoren, Angiogenese-Inhibitoren, Gewebeplasminogen-Aktivatoren, Blutgerinnungs-faktoren, Trypsin-Inhibitoren, Elastase-Inhibitoren, Komplementbestandteile, hypoxie-induzierte Stressproteine, Proto-Oncogen-Produkte, Transkriptionsfaktoren, viruskonstitutive Proteine, Proinsulin, Prourokinase, Erythropoietin, Thrombopoietin, Neurotrophin, Protein C, Glucocerebrosidase, Superoxid-Dismutase, Renin, Lysozym, P450, Prochymosin, Lipocortin, Reptin, Serumalbumin, Streptokinase, Tenecteplase, CNTF und Cyclodextrin-Glycosyl-Transferasen.

Beispiele für von molekularen Scaffolds abgeleitete Proteine sind u.a. Evibodies (abgeleitet von CTLA-4), Affibodies (Protein A von *S. aureus*), Avimere (von humaner A-Domäne Familie), Transbodies (von Transferrin), DARPins (vom Ankyrin Repeat Protein), Adnectin (von Fibronectin III), Peptid Aptamere (von Thioredoxin), Microbodies (von Microprotein), Affiline (von Ubiquitin), $\alpha$-Crystallin, Charybdotoxin, Tetranectin, PDZ Domäne des RAS-bindenden Proteins AF-6, Kunitz-Typ Domäne von Proteininhibitoren.

Im Rahmen dieser Erfindung wird ein eventuell verwendeter Selektionsmarker nicht als Gen codierend für ein rekombinantes Protein betrachtet. Der Begriff Selektionsmarker bezeichnet ein Gen, das als Marker zusammen mit dem "gene of interest", also dem eigentlich gewünschten Gen codierend das rekombinante Protein, in den veränderten Organismus eingebracht wird, um Individuen mit erfolgreicher Genveränderung erkennen zu können. Häufig verwendete Selektionsmarker sind Antibiotikaresistenzen oder Auxotrophien. Die Markergene verschaffen einer Zelle bzw. einem Organismus unter bestimmten Bedingungen einen Überlebensvorteil, so ermöglicht beispielsweise die Expression von $\beta$-Lactamase vom Ampicillin-Resistenzgen als Markergen der Zelle das Überleben in Ampicillin-haltigem Medium. Rekombinante Proteine werden dagegen mit Hilfe des Bakterienstammes produziert und nach einer gewissen Kulturdauer isoliert.

Im Vergleich zur chromosomalen Integration der ORFs codierend das rekombinante Protein und die Peptidoglykan-Peptidase liegt der Vorteil der Verwendung von Plasmiden darin, dass die Plasmid-tragenden Zellen des Bakterienstamms einen Selektionsvorteil haben und durch Standardmethoden selektiert werden können. Darüber hinaus ist es von besonderem Vorteil, dass Plasmide in den Zellen des Bakterienstamms in hoher Kopienzahl vorliegen können.

Gegenstand der Erfindung ist ein Verfahren zur fermentativen Produktion von rekombinanten Proteinen, das dadurch gekennzeichnet ist, dass ein Bakterienstamm in einem Fermentationsmedium kultiviert, das Fermentationsmedium nach der Fermentation von den Zellen abgetrennt und rekombinante Proteine aus dem Fermentationsmedium isoliert werden.

Die Kultivierung der durch chromosomale Integration oder mit einem oder zwei Expressionsplasmiden transformierten Zellen des Bakterienstamms erfolgt nach üblichen, dem Fachmann bekannten Verfahren im Schüttelkolben, oder in einem Bioreaktor (Fermenter).

Als Fermentationsmedien (Nährmedien, Kulturmedien) kommen im Prinzip alle gängigen, dem Fachmann bekannten Medien für die Kultivierung von Bakterien in Frage. Dabei können Komplexmedien oder Minimalsalzmedien, denen ein bestimmter Anteil von Komplex-Komponenten, wie z.B. Pepton, Trypton, Hefeextrakt, Melasse oder Corn Steep liquor zugesetzt wird, benutzt werden. Des Weiteren können dem Medium weitere Komponenten zugegeben werden wie Vitamine, Salze, Aminosäuren und Spurenelemente, durch die das Zellwachstum verbessert wird.

Die Fermentation findet vorzugsweise in einem üblichen Bioreaktor, beispielsweise einem Rührkessel, einem Blasensäulen-Fermenter oder einem Airlift-Fermenter statt. Besonders bevorzugt ist ein Rührkessel-Fermenter.

Bei der Fermentation werden die Zellen des Protein-Produktionsstammes in einem Nährmedium kultiviert, wobei verschiedene Parameter, wie z.B. die Nährstoff-Zufuhr, der Sauerstoff-Partialdruck, der pH-Wert und die Temperatur der Kultur laufend kontrolliert und genau gesteuert werden. Der Zeitraum der Kultivierung beträgt vorzugsweise 24 - 65 h.

Als primäre Kohlenstoffquelle für die Fermentation können prinzipiell alle von den Zellen verwertbaren Zucker, Zuckeralkohole oder organische Säuren bzw. deren Salze verwendet werden. Dabei werden bevorzugt Glukose, Laktose, Arabinose oder Glycerin eingesetzt. Besonders bevorzugt sind Glukose und Arabinose. Auch ist eine kombinierte Fütterung mehrerer verschiedener Kohlenstoffquellen möglich. Die Kohlenstoffquelle kann dabei zu Beginn der Fermentation vollständig im Fermentationsmedium vorgelegt werden oder es wird nichts oder nur ein Teil der Kohlenstoffquelle zu Beginn vorgelegt und die Kohlenstoffquelle wird über den Verlauf der Fermentation zugefüttert. Besonders bevorzugt ist dabei eine Ausführungsform, bei der ein Teil der Kohlenstoffquelle vorgelegt wird und ein Teil gefüttert wird. Besonders bevorzugt wird die Kohlenstoffquelle Glukose in einer Konzentration von 10 - 30 g/l vorgelegt, die Fütterung gestartet, wenn die Konzentration im Verlauf der Fermentation auf kleiner 5 g/l abgefallen ist und so gestaltet, dass die Konzentration unter 5 g/l gehalten wird.

Wird die Expression des rekombinanten Proteins und/oder der Peptidoglykan-Peptidase durch einen induzierbaren Promotor kontrolliert, so erfolgt die Induktion der Expression durch Zugabe des zum Promotor passenden Induktors zum Fermentationsansatz. Als Induktor eignen sich beispielsweise Arabinose, Laktose, IPTG, Tetracyclin oder Cumate. Der Induktor kann zu einem beliebigen Zeitpunkt während der Fermentation als Einmal- oder Mehrfach-Gabe zudosiert werden. Alternativ kann der Induktor kontinuierlich zugegeben werden. Bevorzugt wird die Expression des rekombinanten Proteins durch Zugabe von IPTG und die Expression der Peptidoglykan-Peptidase durch Zugabe von Arabinose induziert. Besonders bevorzugt wird IPTG als Einmalgabe zudosiert. Die Induktion der Expression der Peptidoglykan-Peptidase wird bevorzugt so gestaltet, dass zu einem Zeitpunkt nach Abfall der Glukosekonzentration unter 5 g/l ein Gemisch aus Glukose und Arabinose zugefüttert wird, oder die Arabinose wird als Einmalgabe zudosiert.

Die Induktion der Expression der Peptidoglykan-Peptidase erfolgt bevorzugt in der Phase der Kultivierung, in der nur noch wenige bis keine Zellteilungen mehr erfolgen, d.h. kurz vor bzw. am Anfang der stationären Phase der Wachstumskurve. Dieser Zeitpunkt wird dadurch bestimmt, dass die Zelldichte bestimmt als $OD_{600}$- oder CDW-Wert (s.u.) nur noch wenig bis gar nicht mehr ansteigt.

Vorzugsweise wird das Medium im Fermenter vor Inokulation gerührt und mit entkeimter Druckluft begast, dabei wird der Sauerstoff-Gehalt auf 100% Sättigung kalibriert und ein Soll-Wert für die $O_2$-Sättigung während der Fermentation gewählt, der zwischen 10 und 70%, bevorzugt zwischen 20 und 60% und besonders bevorzugt bei 30% dieses Werts liegt. Nach Absinken der $O_2$-Sättigung unter den Soll-Wert startet eine Regulationskaskade, um die $O_2$-Sättigung wieder an den Soll-Wert heranzuführen, wobei Gaszufuhr und Rührgeschwindigkeit angepasst werden können.

Der pH-Wert der Kultur liegt vorzugsweise zwischen pH 6 und pH 8. Bevorzugt wird ein pH-Wert zwischen 6,5 und 7,5 eingestellt, besonders bevorzugt wird der pH-Wert der Kultur zwischen 6,8 und 7,2 gehalten.

Die Temperatur der Kultur beträgt vorzugsweise zwischen 15 und 45 °C. Bevorzugt ist ein Temperaturbereich zwischen 20 und 40 °C, besonders bevorzugt ist ein Temperaturbereich zwischen 25 und 35 °C, ganz besonders bevorzugt beträgt die Temperatur 30 °C.

Erfindungsgemäß wird das Fermentationsmedium nach der Fermentation von den Zellen abgetrennt und rekombinante Proteine werden aus dem Fermentationsmedium isoliert. Dies kann durch übliche Methoden geschehen, wie sie im Stand der Technik bekannt sind. Üblicherweise werden in einem ersten Schritt durch Separationsmethoden wie Zentrifugation oder Filtration die Zellen von den in das Kulturmedium freigesetzten rekombinanten Proteinen abgetrennt. Die rekombinanten Proteine können dann z.B. durch Ultrafiltration aufkonzentriert werden.

Durch die Expression der Peptidoglykan-Peptidase wird die Freisetzung von rekombinanten Proteinen in den Kulturüberstand verbessert. Durch die verbesserte Freisetzung lassen sich rekombinante Proteine in erhöhter Ausbeute isolieren.

Für den Begriff "erhöhte Ausbeute" gilt obige Definition.

Mit Hilfe der Coexpression der Peptidoglykan-Peptidase in einem Bakterienstamm, bevorzugt in *E. coli,* können auch Fab-Antikörperfragmente extrazellulär hergestellt werden. Dabei muss die Bakterienzelle die entsprechenden Fragmente der leichten Kette, welche die Domänen VL und CL umfasst, und der schweren Kette, welche die Domänen VH und CH1 umfasst, des Antikörpers gleichzeitig synthetisieren und dann in das Periplasma sekretieren. Außerhalb des Cytoplasmas erfolgt dann die Assemblierung der beiden Ketten zum funktionellen Fab-Fragment. Die entsprechenden ORFs können in verschiedenen Genen liegen. Es ist bevorzugt, dass die ORFs codierend Antikörperfragmente der leichten und schweren Kette in einem Operon organisiert sind. Durch gleichzeitige Produktion der erfindungsgemäßen Peptidoglykan-Peptidase werden die schwere und die leichte Kette des Antikörpers in erhöhter Konzentration in das Fermentationsmedium freigesetzt.

Das Verfahren hat den großen Vorteil, dass es zur Produktion rekombinanter Proteine, die eine lange Kulturdauer verlangen, sowie für die Fermentation bei hohen Zelldichten von > 40 g/l geeignet ist. Dadurch lassen sich komplexe, eukaryontische Proteine als rekombinante Proteine herstellen.

Bevorzugt ist das Verfahren dadurch gekennzeichnet, dass nach dem Abtrennen des Fermentationsmediums die rekombinanten Proteine aus dem Fermentationsmedium aufgereinigt werden.

Rekombinante Proteine können über Standardmethoden wie Fällung oder Chromatographie weiter gereinigt werden. Besonders bevorzugt sind hierbei Methoden, wie Affinitätschromatographie, bei der die bereits korrekt gefaltete native Konformation des Proteins ausgenutzt wird.

Bevorzugt ist das Verfahren dadurch gekennzeichnet, dass die Expression der Peptidoglykan-Peptidase induziert wird. Das bedeutet, dass entweder nur die Expression der Peptidoglykan-Peptidase oder die Expression des rekombinanten Proteins und die Expression der Peptidoglykan-Peptidase induziert wird. Das heißt, dass in dieser bevorzugten Ausführungsform die Expression der Peptidoglykan-Peptidase in jedem Fall unter Kontrolle eines induzierbaren Promotors steht. Dagegen kann die Expression des rekombinanten Proteins unter Kontrolle eines konstitutiven oder eines induzierbaren Promotors stehen. Bevorzugt stehen sowohl der ORF codierend das Signalpeptid und das rekombinante Protein, als auch der ORF codierend das Signalpeptid und die Peptidoglykan-Peptidase unter Kontrolle eines induzierbaren Promotors, besonders bevorzugt von unterschiedlich induzierbaren Promotoren. Bevorzugt ist daher die Expression der Peptidoglykan-Peptidase und die des rekombinanten Proteins induzierbar.

Da die Promotoren der Gene codierend das rekombinante Protein und die Peptidoglykan-Peptidase bevorzugt unabhängig voneinander induziert werden können, kann für die Expression des rekombinanten Proteins und für die Expression der Peptidoglykan-Peptidase unabhängig voneinander der optimale Zeitpunkt gewählt werden.

Durch die Verwendung induzierbarer Promotoren kann die Expression der entsprechenden Proteine zu einem beliebigen Zeitpunkt der Fermentation induziert werden. Bevorzugt wird die Expression der Peptidoglykan-Peptidase nach der Induktion der Expression des rekombinanten Proteins induziert. Besonders bevorzugt wird die Expression der Peptidoglykan-Peptidase mindestens 1 Stunde, insbesondere bevorzugt mindestens 2 Stunden, darüber hinaus besonders bevorzugt 15 bis 40 Stunden und im speziellen bevorzugt 20, 27 oder 38 Stunden nach der Induktion der Expression des rekombinanten Proteins induziert.

Die Induktion der Expression des rekombinanten Proteins sowie der Peptidoglykan-Peptidase wird durch Zugabe des Induktors zum Kulturmedium ausgelöst, wobei der Induktor entsprechend des verwendeten Gens gewählt werden muss. In der bevorzugten Ausführungsform, in der die Peptidoglykan-Peptidase unter Kontrolle des Arabinose (ara)-Promotor steht, wird die Expression der Peptidoglykan-Peptidase durch Zugabe des Induktors Arabinose zur Kultur induziert. In der bevorzugten Ausführungsform, in der das rekombinante Gen einen tac-Promotor enthält, wird die Expression des rekombinanten Proteins durch Zugabe des Induktors Laktose oder des Laktose-Analogons IPTG zur Kultur induziert.

Erfindungsgemäß ist auch nach Induktion der Expression der Peptidoglykan-Peptidase und weiterer Kultur der Zellen die Zelldichte des Stammes vergleichbar zu einem Bakterienstamm, der keine zusätzliche Peptidoglykan-Peptidase exprimiert. Insbesondere kommt es nicht zu einer starken Abnahme der Zelltrockenmasse bedingt durch Zelllyse.

Die Erfindung hat den Vorteil, dass rekombinante Proteine in das Kulturmedium freigesetzt werden, ohne dass es zu starker Lyse der Zellen des Bakterienstamms kommt. Das erfindungsgemäße Verfahren zeichnet sich daher dadurch aus, dass es sowohl zur Produktion komplexer, eukaryontischer Proteine mit entsprechend langer Kulturdauer, als auch für die Fermentation bei hohen Zelldichten >40 g/l geeignet ist.

Eine lange Kulturdauer bedeutet, dass die Kultivierungszeit mindestens 24 h, bevorzugt mindestens 48 h und besonders bevorzugt mindestens 65 h beträgt.

Eine geringere (bzw. nur gering erhöhte oder keine starke) Zelllyse bedeutet im Rahmen der Erfindung, dass der CDW-Wert, $OD_{600}$- Wert oder der Wert für die Lebendzellzahl der Kultur des Bakterienstammes, die bei einer Kulturdauer von vorzugsweise 7-17 h nach Induktion der Expression der Peptidoglykan-Peptidase im Vergleich zum Wildtyp-Bakterienstamm, der keine zusätzliche Peptidoglykan-Peptidase produziert, bestimmt wird, nur wenig bis gar nicht verringert ist. Insbesondere zur Kultur von Bakterienstämmen exprimierend Peptidoglykan-Glykosidasen, deren Zelllyse wie von Morita et al. (2001, s.o.) und in den Patentschriften CN 104762285 und EP 1 124 941 für Zellen exprimierend T4-Phagen-Lysozym bzw. das ClyN-Protein gezeigt, massiv erhöht ist, haben die Bakterienstämme exprimierend eine Peptidoglykan-Peptidase einen großen Vorteil.

Bevorzugt ist das Verfahren dadurch gekennzeichnet, dass sich 5 bis 24 Stunden nach Induktion der Expression der zusätzlichen Peptidoglykan-Peptidase die Zelltrockenmasse um höchstens 20% von der Zelltrockenmasse einer Zellkultur zum gleichen Zeitpunkt aus einem Fermentationsverfahren eines Bakterienstamms, der die zusätzliche Peptidoglykan-Peptidase nicht exprimiert, unterscheidet. Bevorzugt ist eine Zellkultur aus dem erfindungsgemäßen Fermentationsverfahren dadurch gekennzeichnet, dass sie 5 bis 24 Stunden, besonders bevorzugt 7 Stunden oder 17 Stunden nach Induktion der Expression der zusätzlichen Peptidoglykan-Peptidase eine Zelltrockenmasse aufweist, die um höchstens 20%, besonders bevorzugt höchstens 15% und insbesondere bevorzugt höchstens 10% geringer ist, als die Zelltrockenmasse einer Zellkultur zum gleichen Zeitpunkt aus einem Fermentationsverfahren welches sich vom erfindungsgemäßen Verfahren nur dadurch unterscheidet, dass ein Bakterienstamm kultiviert wird, der sich vom erfindungsgemäßen Bakterienstamm nur dadurch unterscheidet, dass er den ORF codierend eine Peptidoglykan-Peptidase nicht enthält. Die Zelltrockenmasse dient als Maß der Zelldichte und damit der nicht lysierten Zellen. Die Zelltrockenmasse wird bestimmt, indem eine definierte Kulturmenge durch Filtrieren oder Zentrifugieren, bevorzugt Zentrifugieren, isoliert, anschließend getrocknet und gewogen wird.

Fig. 1 zeigt die Plasmidkarte des Plasmids pCGT-Spr.
Fig. 2 zeigt die Plasmidkarte des Plasmids pCGT-YdhO.
Fig. 3 zeigt die Plasmidkarte des Plasmids pJF118ut-CD154.
Fig. 4 zeigt die Plasmidkarte des Plasmids pJF118ut-CD154-Spr.
Fig. 5 zeigt die Analyse der Kulturüberstände durch SDS-PAGE, wie in Beispiel 1 beschrieben. Es gilt folgende Probenbezeichnung: S: SeeBlue Prestained Protein-Standard, 1: Überstand von W3110/pCGT nach 65 h, 2: Überstand von W3110/pCGT-Spr ohne Arabinose-Zugabe nach 65 h, 3: Überstand von W3110/pCGT-Spr mit Arabinose-Zugabe nach 65 h.

Die in den Figuren verwendeten Abkürzungen stehen für DNS-Regionen, die die folgenden Funktionen codieren:

| | |
|---|---|
| tac p/o: | tac-Promotor/Operator |
| pBAD p/o: | Arabinose-Promotor/Operator |
| bla: | β-Lactamase-Gen (Ampicillin-Resistenz) |
| TcR: | Tetracyclin-Resistenz |
| lacIq: | Repressor des tac-Promotors |
| cgt-SP: | Signalpeptid der CGTase |
| CGTase: | Cyclodextrin-Glycosyl-Transferase |
| ColE1: | Replikationsursprung |
| phoA-SP: | phoA-Signalpeptid |
| AFA-SP: | Derivat des Signalpeptids der CGTase |
| rrnB-Terminator: | Terminatorregion des rrnB-Gens |
| trpA-Terminator: | Terminatorregion des trpA-Gens |
| Spr: | Peptidoglykan-Peptidase Spr |
| Spr-SP: | Signalpeptid von Spr |
| YdhO: | Peptidoglykan-Peptidase YdhO |
| HisTag: | Histidin-Tag |
| light chain: | Antikörperfragment umfassend die Domänen VL und CL |
| heavy chain: | Antikörperfragment umfassend die Domänen VH und CH1 |
| BamHI/MauBI/EcoRI: | Schnittstellen der entsprechenden Restriktionsendonukleasen |

**Beispiele**

Die Erfindung wird im Folgenden anhand von Ausführungsbeispielen näher beschrieben, ohne dadurch beschränkt zu werden.

Sämtliche eingesetzten molekularbiologischen Verfahren, wie Polymerase-Ketten-Reaktion (PCR), Gensynthese, Isolierung und Reinigung von DNS, Modifikation von DNS durch Restriktionsenzyme und Ligase, Transformation usw. wurden in der dem Fachmann bekannten, in der Literatur beschriebenen oder von den jeweiligen Herstellern empfohlenen Art und Weise durchgeführt.

**Beschreibung der Plasmide:**

pCGT:

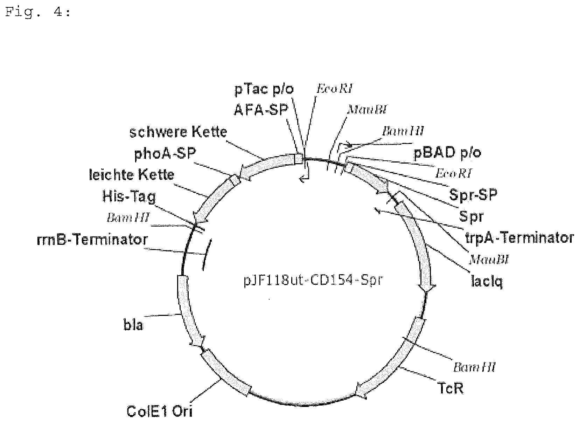

Die Herstellung des Plasmids pCGT ist in Beispiel 4 von US 2008/0254511 A1 beschrieben, die Plasmidkarte in Fig. 4 von US 2008/0254511 A1 angegeben.

Im Wesentlichen enthält das Plasmid neben dem Gen für die Resistenz gegen Tetracyclin u.a. auch das Strukturgen der Cyclodextrin-Glycosyl-Transferase (CGTase) aus *Klebsiella pneumoniae* M5a1 einschließlich der nativen CGTase-Signalsequenz. Die Expression des CGTase-Gens steht unter der Kontrolle des tac-Promotors.

pCGT-Spr:

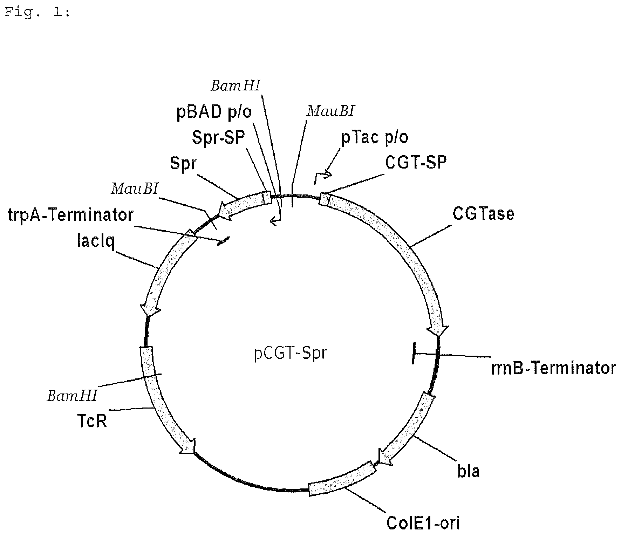

Um pCGT-Spr (Plasmidkarte s. Fig. 1) zu erhalten, wurde ein DNS-Fragment mittels Gensynthese von der Firma Eurofins Genomics hergestellt. Dieses DNS-Fragment xl (angegeben in SEQ ID Nr. 1) enthielt:

- die Nukleotide 1136-1304 aus GenBank-Eintrag X81837.1, enthaltend den Arabinose-Promotor (pBAD-Promotor), sowie die Operatoren O1 und I2+I1 und die CAP-Bindestelle (Nukleotide 10-178 von SEQ ID Nr. 1),
- die Shine-Dalgarno-Sequenz (Nukleotide 203-208 von SEQ ID Nr. 1) und
- ein Nukleotid-Fragment, codierend für eine Fusion aus

> i dem Spr-Protein aus *E. coli* K12 (Nukleotide 216-782 von SEQ ID Nr. 1) und
> ii dem Terminator des trpA-Gens aus *E. coli* (Nukleotide 812-837 von SEQ ID Nr. 1).

Dieses DNS-Fragment xI wurde mit dem Restriktionsenzym MauBI geschnitten und mit dem Expressionsvektor pCGT, der mit demselben Restriktionsenzym geschnitten worden war, ligiert. Die Klonierung erfolgte ungerichtet, es wurde jedoch mit dem Plasmid gearbeitet, in dem das DNS-Fragment in der gleichen Leserichtung wie das Gen codierend für die CGTase eingefügt war, wobei der Nachweis über das Restriktionsmuster des Restriktionsenzyms BamHI und Sequenzieren erfolgte. Dieses Plasmid wurde als pCGT-Spr bezeichnet und codiert das Spr-Protein.

pCGT-YdhO:

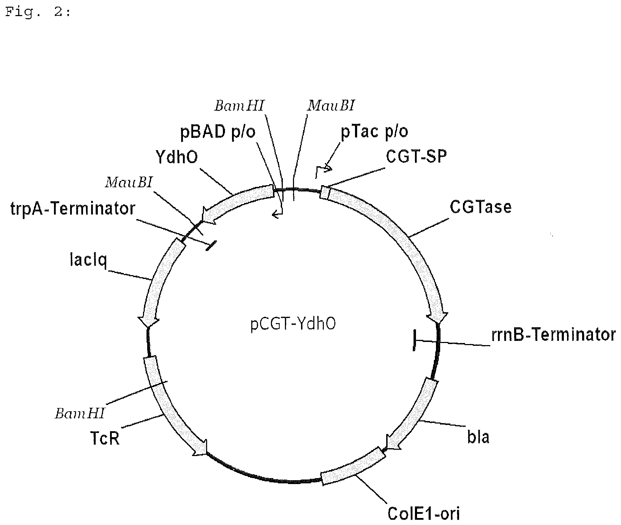

Um pCGT-YdhO (Plasmidkarte s. Fig. 2) zu erhalten, wurde ein DNS-Fragment mittels Gensynthese von der Firma Eurofins Genomics hergestellt. Dieses DNS-Fragment xII (angegeben in SEQ ID Nr. 2) enthielt:

- die Nukleotide 1136-1304 aus GenBank-Eintrag X81837.1, enthaltend den Arabinose-Promotor (pBAD-Promotor), sowie die Operatoren O1 und I2+I1 und die CAP-Bindestelle (Nukleotide 10-178 von SEQ ID Nr. 2),
- die Shine-Dalgarno-Sequenz (Nukleotide 203-208 von SEQ ID Nr. 2) und
- ein Nukleotid-Fragment, codierend für eine Fusion aus

> i dem YdhO-Protein aus *E. coli* K12 (Nukleotide 216-1028 von SEQ ID Nr. 2) und
> ii dem Terminator des trpA-Gens aus *E. coli* (Nukleotide 1064-1089 von SEQ ID Nr. 2).

Dieses DNS-Fragment xII wurde mit dem Restriktionsenzym MauBI geschnitten und mit dem Expressionsvektor pCGT, der mit demselben Restriktionsenzym geschnitten worden war, ligiert. Die Klonierung erfolgte ungerichtet, es wurde jedoch mit dem Plasmid gearbeitet, in dem das DNS-Fragment in der gleichen Leserichtung wie das Gen codierend für die CGTase eingefügt war, wobei der Nachweis über das Restriktionsmuster des Restriktionsenzyms BamHI und Sequenzieren erfolgte. Dieses Plasmid wurde als pCGT-YdhO bezeichnet und codiert das YdhO-Protein.

pJF118ut-CD154:

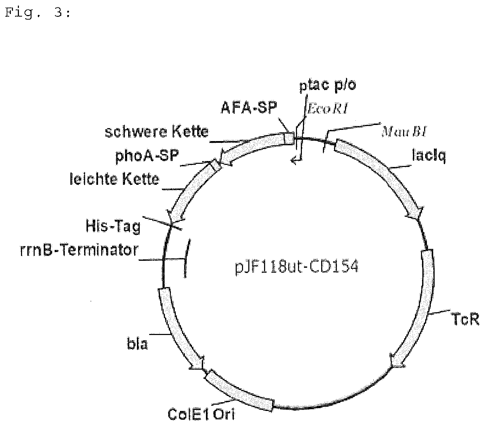

Als Ausgangsvektor für die Klonierung und Expression der Gene des Fab-Fragments des humanisierten monoklonalen anti-CD154-Antikörpers 5c8, dessen Sequenz in Karpusas et al. 2001 (Structure 9, S. 321-329) veröffentlicht ist, diente das in US 2008/076157 A beschriebene Plasmid pJF118ut. pJF118ut ist ein Derivat des bekannten Expressionsvektors pKK223-3 (Amersham Pharmacia Biotech) und bei der DSMZ-Deutsche Sammlung von Mikroorganismen und Zellkulturen GmbH (Braunschweig) unter der Nummer DSM 18596 hinterlegt.
Um pJF118ut-CD154 (Plasmidkarte s. Fig. 3) zu erhalten, wurde ein DNS-Fragment per Gensynthese von der Firma eurofins Genomics hergestellt. Dieses DNS-Fragment xIII (angegeben in SEQ ID Nr. 3) umfasste eine Fusion bestehend aus:

> i der in US 2008/076157 unter der SEQ ID Nr. 2 offenbarten Signalsequenz, abgeleitet aus der Signalsequenz einer CGTase aus *Klebsiella pneumoniae* M5a1 (Nukleotide 25-114 von SEQ ID Nr. 3) und
> ii dem Leserahmen für die schwere Kette (V$_H$-C$_H$1-Domänen) des Fab-Fragments des humanisierten monoklonalen anti-CD154-Antikörpers 5c8, codierend die Aminosäuren 1-221 der in Karpusas et al. 2001 in Fig. 3 veröffentlichten Sequenz (Nukleotide 115-777 von SEQ ID Nr. 3),
> iii der phoA-Signalsequenz (Nukleotide 800-862 von SEQ ID Nr. 3),
> iv dem Leserahmen für die leichte Kette (V$_L$-C$_L$-Domänen) des Fab-Fragments des humanisierten monoklonalen anti-CD154-Antikörpers 5c8, wie von Karpusas et al. 2001 in Fig. 3 veröffentlicht (Nukleotide 863-1516 von SEQ ID Nr. 3) und
> v den Nukleotiden 1517-1546 von SEQ ID Nr. 3, codierend einen 4-Aminosäure-langen Linker und einen Hexa-Histidin-Tag.

Dieses DNS-Fragment xIII wurde mit den Restriktionsenzymen EcoRI und PdmI geschnitten und mit dem Expressionsvektor pJF118ut, der mit EcoRI und SmaI geschnitten worden war, ligiert. Das resultierende Plasmid, bei dem die Expression der Gene für die schwere und leichte Kette des Fab-Fragments unter der Kontrolle des tac-Promotors standen, wurde mit pJF118ut-CD154 bezeichnet.

pJF118ut-CD154-Spr:

In das Plasmid pJF118ut-CD154 wurde das DNS-Fragment xI codierend für Spr unter Kontrolle des Arabinose-Promotors und flankiert vom trpA-Terminator, wie für pCGT-Spr oben beschrieben, über die MauBI Schnittstelle eingeführt. Die Klonierung erfolgte ungerichtet, es wurde jedoch mit dem Plasmid gearbeitet, in dem das DNS-Fragment xI in der entgegengesetzten Leserichtung wie das DNS-Fragment xIII codierend für CD154 eingefügt war, wobei der Nachweis über das Restriktionsmuster des Restriktionsenzyms BamHI und Sequenzieren erfolgte. Dieses Plasmid wurde als pJF118ut-CD154-Spr bezeichnet (Plasmidkarte s. Fig. 4).

**Beispiel 1:** Fermentative Produktion der Cyclodextrin-Glycosyl-Transferase (CGTase) im Rührkesselfermenter

Für die Produktion der Cyclodextrin-Glycosyl-Transferase (CGTase) aus Klebsiella pneumoniae M5a1 wurde der *E. coli*-Stamm W3110 (ATCC 27325) mit den Plasmiden pCGT, pCGT-Spr und pCGT-YdhO nach gängigen Methoden (z.B. TSS-Transformation) transformiert. Die Selektion auf plasmidhaltige Zellen erfolgte mittels Tetracyclin (20 mg/l). Die *E. coli*-Stämme wurden mit W3110/pCGT, W3110/pCGT-Spr und W3110/pCGT-YdhO bezeichnet.

a) Die CGTase-Produktion erfolgte in Rührkesselfermentern.

Der mit 1,2 l des Fermentationsmediums (1,5 g/l $KH_2PO_4$; 5 g/l $(NH_4)_2SO_4$; 0,5 g/l $MgSO_4 \times 7\ H_2O$; 0,225 g/l $CaCl_2 \times 2\ H_2O$, 0,075 g/l $FeSO_4 \times 7\ H_2O$; 1 g/l $Na_3Citrat \times 2\ H_2O$; 0,5 g/l NaCl; 1 ml/l Spurenelementlösung (0,15 g/l $Na_2MoO_4 \times 2\ H_2O$; 2,5 g/l $Na_3BO_3$; 0,7 g/l $CoCl_2 \times 6\ H_2O$; 0,25 g/l $CuSO_4 \times 5\ H_2O$; 1,6 g/l $MnCl_2 \times 4\ H_2O$; 0,3 g/l $ZnSO_4 \times 7\ H_2O$); 5 mg/l Vitamin B1; 3 g/l Phyton-Pepton (BD 211906); 1,5 g/l Hefeextrakt (Oxoid LP0021); 10 g/l Glukose; 20 mg/l Tetracyclin) befüllte Fermenter wurde ad 0,1 $OD_{600}$ mit einer Vorkultur von W3110/pCGT bzw. W3110/pCGT-Spr, die für 7 h im Schüttelkolben in LB-Medium (5 g/l Hefeextrakt (Oxoid LP0021), 10 g/l Trypton (Oxoid LP0042), 5 g/l NaCl), zusätzlich enthaltend 1 ml/l Spurenelementlösung (0,15 g/l Na2MoO4 $\times$ 2 H2O; 2,5 g/l Na3BO3; 0,7 g/l CoCl2 $\times$ 6 H2O; 0,25 g/l CuSO4 $\times$ 5 H2O; 1,6 g/l MnCl2 $\times$ 4 H2O; 0,3 g/l ZnSO4 $\times$ 7 H2O), 3 g/l Glukose, 0,55 g/l $CaCl_2$ und 20 mg/l Tetracyclin kultiviert worden waren, angeimpft. Damit wurde die Fermentation gestartet (Zeitpunkt 0, Fermentationsbeginn). Während der Fermentation wurde eine Temperatur von 30 °C eingestellt und der pH-Wert durch Zudosierung von $NH_4OH$ bzw. $H_3PO_4$ bei einem Wert von 7,0 konstant gehalten. Glukose wurde über die Fermentation hinweg zudosiert, wobei eine Glukose-Konzentration von < 5 g/l angestrebt wurde. Die Induktion der Expression der CGTase erfolgte durch Zugabe von Isopropyl-β-D-thiogalactopyranosid (IPTG) ad 0,15 mM nach 21 h (am Ende der logarithmischen Wachstumsphase). Für die Spr-Produktion wurde die Basalexpression des Arabinose-Promotors genutzt, die im Laufe der Fermentation zur Bildung geringer Mengen des Spr-Proteins führt. Dass der Arabinose-Promotor auch in Abwesenheit von Induktor eine geringe Produktion des Zielproteins ermöglicht, ist im Stand der Technik beschrieben (Malachowska and Olszewski, Microb Cell Fact (2018) 17:40).

Das Medium im Fermenter wurde vor Inokulation mit 400 rpm gerührt und mit 1,67 vvm (Volumen Luft pro Volumen Kulturmedium pro Minute) einer über einen Sterilfilter entkeimten Druckluft begast. Unter diesen Startbedingungen wurde der optische Sauerstoff-Sensor (VisiFerm DO225, Hamilton) vor der Inokulation auf 100% Sättigung kalibriert. Der Soll-Wert für die $O_2$-Sättigung während der Fermentation wurde auf 30 % dieses Werts eingestellt. Die $O_2$-Sättigung wurde während der Fermentation über den Sauerstoffsensor gemessen und von der Fermenter-Steuerung DCU (Digital control unit, Sartorius Stedim) erfasst. Nach Absinken der $O_2$-Sättigung unter den Soll-Wert wurde Software-gesteuert die Rührgeschwindigkeit kontinuierlich auf max. 1.500 rpm gesteigert, um die $O_2$-Sättigung wieder an den Soll-Wert heranzuführen.

Nach 65 h Fermentation wurden Proben entnommen, die Zellen durch Zentrifugation vom Fermentationsmedium abgetrennt und der CGTase-Gehalt im Fermentationsüberstand durch folgenden Enzymtest bestimmt:

| | |
|---|---|
| Testpuffer: | 5 mM Tris-HCl-Puffer, 5 mM $CaCl_2$ x 2 $H_2O$, pH 6,5 |
| Substratlösung: | 10 %ige Stärkelösung (Merck Nr.1.01252) in Testpuffer, pH 6,5 |
| Testansatz: | 0,2 ml Substratlösung + 0,2 ml abzentrifugierter (5 min, 12 000 rpm) Kulturüberstand |
| Reaktionstemperatur: | 40°C |

Enzymtest:

* Vortemperieren von Substratlösung und abzentrifugiertem Kulturüberstand (ca. 5 min bei 40°C)

* Herstellen des Testansatzes durch rasches Mischen (Whirl-Mixer) von Substratlösung und abzentrifugiertem Kulturüberstand, wobei der abzentrifugierte Kulturüberstand gegebenenfalls mit Testpuffer verdünnt wird, so dass in der anschließenden HPLC-Analytik ein Wert von 0,9-1,5 g/l CD bestimmt wird;

* Inkubation für 3 min bei 40°C

* Stoppen der Enzymreaktion durch Zugabe von 0,6 ml Methanol, rasches Mischen (Whirl-Mixer)

* Abkühlen des Ansatzes auf Eis (ca. 5 min)

* Abzentrifugieren (5 min, 12 000 rpm) und Abpipettieren des klaren Überstandes

* Analyse der Menge an produziertem CD mittels HPLC: Die Analyse wurde an einem Agilent HP 1100 HPLC-System mit einer Nucleodur 100-3 NH2-RP Säule (150 mm x 4,6 mm, Macherey-Nagel) und 64% Acetonitril in Wasser(v/v) als Fließmittel, bei einer Flussrate von 2,1 ml/min durchgeführt. Die Detektion erfolgte über einen RI-Detektor (1260 Infinity RI, Agilent) und die Quantifizierung wurde anhand der Peakfläche und einem $\alpha$-CD-Standard (Cavamax W6-8 Pharma, Wacker Chemie AG) vorgenommen.

Berechnung der Enzymaktivität:    $A = G*V1*V2/(t*MG)$ [U/ml]

A =    Aktivität,
G =    Gehalt an CD in mg/l
V1 =    Verdünnungsfaktor im Testansatz
V2 =    Verdünnungsfaktor des Kulturüberstands vor dem Einsatz im Test; wenn unverdünnt gilt: V2 = 1
t =    Reaktionszeit in min
MG =    Molekulargewicht in g/mol ($MG_{CD}$ = 973 g/mol)
1 Unit (U)    $\hat{=}$ 1 $\mu$mol/l Produkt (CD) / min

Tabelle 1 zeigt die jeweils erzielten Cyclodextrin-Glycosyltransferase-Ausbeuten.

**Tabelle 1:** CGTase-Ausbeuten im Fermentationsüberstand nach 65 h Kultivierung.

| Stamm | CGTase (U/ml) |
|---|---|
| W3110/pCGT | 31 |
| W3110/pCGT-Spr | 295 |

Um zu untersuchen, ob die Freisetzung von CGTase durch Produktion von Spr auf eine verstärkte Zelllyse zurückzuführen ist, wurde am Ende der Fermentation die Zelltrockenmasse und die Lebendzellzahl (LZZ) der Kulturen bestimmt. Zur Bestimmung der Lebendzellzahl wurden Proben der Kulturen $10^8$-fach mit LB-Medium in einem Endvolumen von 1 ml verdünnt und jeweils 100 $\mu$l der Verdünnung auf LB-Agarplatten enthaltend 20 mg/l Tetracyclin ausplattiert. Die gewachsenen Kolonien wurden ausgezählt und unter Berücksichtigung des Verdünnungsfaktors die Lebendzellzahl der Ausgangskulturen zu $50 \cdot 10^9$ für W3110/pCGT und zu $39 \cdot 10^9$ für W3110/pCGT-Spr berechnet. Zur Bestimmung der Zelltrockenmasse wurden Proben, enthaltend 1 ml Fermenterkultur, in Reaktionsgefäße transferiert, deren Leergewicht zuvor bestimmt worden war. Nach Zentrifugation (5 min, 12.000 rpm) wurden die Überstände entfernt und die Zellpellets in einem Inkubator getrocknet ($\geq$ 48 h bei 60°C). Anschließend wurden die Gefäße mit dem getrockneten Zellpellet ausgewogen und die Zelltrockenmasse (cell dry weight, CDW) aus der Differenz der Masse der Gefäße mit getrocknetem Zellpellet und der Masse der leeren Gefäße errechnet. Die Zelltrockenmassen betrugen 45 g/l für W3110/pCGT und 55 g/l für W3110/pCGT-Spr.

Eine starke Zelllyse führt zur Freisetzung von Wirtszellprotein. Dies wurde ausgeschlossen, indem der Proteingehalt der Kulturüberstände über SDS-PAGE analysiert wurde (Fig.5, Probe 1 und 2). Nach 65 h Fermentation wurden Proben entnommen und die Zellen durch Zentrifugation vom Fermentationsmedium abgetrennt. Der zellfreie Überstand wurde 1:10 mit Wasser verdünnt, davon 4 $\mu$l mit 4 $\mu$l 2$\times$Tris-Glycine SDS Sample Buffer (Invitrogen, Carlsbad, USA, Cat.No. LC2676) mit 1 ml/l NuPAGE Antioxidant (Invitrogen, Carlsbad, USA, Cat.No. NP0005) und 5% Dithioerythritol versetzt und für 5 min bei 99 °C im Thermoblock aufgekocht. Die Probe wurde 20 s bei 10000 rpm zentrifugiert und jeweils 2 $\mu$l auf ein 12% NuPAGE Bis-Tris-Gel (1.0 mm $\times$ 15 wells, Invitrogen, Carlsbad, USA, Cat.No. NP0343) aufgetragen und die Proteine durch Elektrophorese in 1$\times$MOPS-Puffer (NuPAGE MOPS SDS Running Buffer (20$\times$) Invitrogen, Carlsbad, USA, Cat.No. NP0001; mit Wasser auf 1$\times$ verdünnt) für 3 h bei 95 V aufgetrennt. Als Standard wurde See Blue Prestained (Invitrogen, Carlsbad, USA; Cat.No. 100006636) aufgetragen. Die Proteine wurden mit Instant Blue (Coomassie based staining solution for protein gels, Expedeon Ltd., Cambridge, UK, Cat.No. ISB1L) für 1 h bei Raumtemperatur angefärbt und anschließend über Nacht bei Raumtemperatur in Wasser entfärbt. Im Gel sind neben der Bande des CGTase-Proteins nur geringe Verunreinigungen mit anderen Proteinen zu erkennen. Insbesondere ist keine verstärkte Freisetzung des überproduzierten Spr-Proteins (18 kDa) zu sehen.

b)
Zudem wurde die Produktion der CGTase wie oben beschrieben mit den Stämmen W3110/pCGT-Spr und W3110/pCGT-YdhO durchgeführt. Die Induktion der Produktion von YdhO erfolgte 48 h nach Fermentationsbeginn durch Umstellung der reinen Zufütterung von Glukose auf eine konstante Glukose-Arabinose-Misch-Fütterung von 3 g/l*h im Verhältnis 10:1 Glukose:Arabinose. Die Induktion der Produktion von Spr erfolgte 59 h nach Fermentationsbeginn durch einmalige Zugabe von Arabinose zur Kultur, so dass eine Endkonzentration von 1 g/l erreicht wurde. Die Ernte erfolgte 65 h nach Fermentationsbeginn.

In Tabelle 2 sind die auf diese Weise erzielten CGTase-Ausbeuten gezeigt.

**Tabelle 2:** CGTase-Ausbeuten im Fermentationsüberstand nach 65 h Kultivierung.

| Stamm | CGTase (U/ml) |
|---|---|
| W3110/pCGT-Spr | 1130 |
| W3110/pCGT-YdhO | 1187 |

Wie zuvor beschrieben, wurde nach 65 h Kultivierung die Zelltrockenmasse bestimmt. Es wurden Werte von 50 g/l für W3110/pCGT-Spr und 41 g/l für W3110/pCGT-YdhO erhalten.

Der Kulturüberstand von W3110/pCGT-Spr wurde über SDS-PAGE analysiert und gezeigt, dass neben der Freisetzung von CGTase keine nennenswerte Freisetzung *E. coli*-eigener Proteine erfolgte (Fig. 5, Probe 3).

**Beispiel 2:** Fermentative Produktion eines Fab-Antikörperfragments

Für die Produktion des CD154-Fab-Fragments wurde der *E. coli*-Stamm W3110 mit den Plasmiden pJF118ut-CD154 und pJF118ut-CD154-Spr nach gängigen Methoden (z.B. TSS-Transformation) transformiert. Die Selektion auf plasmidhaltige Zellen erfolgte mittels Tetracyclin (20 mg/l). Die *E. coli*-Stämme wurden mit W3110/pJF118ut-CD154 und W3110/pJF118ut-CD154-Spr bezeichnet.

Die Fermentation dieser *E. coli*-Stämme erfolgte in Rührkesselfermentern, wie für die Fermentation der *E. coli* pCGT-Stämme in Beispiel 1 beschrieben. Im Unterschied zu Beispiel 1 erfolgte die Induktion der CD154-Fab-Produktion durch 0,15 mM IPTG 26 h nach Fermentationsbeginn und die Induktion der Spr-Produktion durch Umstellung der reinen Zufütterung von Glukose auf eine konstante Glukose-Arabinose-Misch-Fütterung von 3 g/l*h im Verhältnis 2:1 Glukose:Arabinose nach 41 h.

Nach 48 h wurden Proben entnommen, die Zellen durch Zentrifugation vom Kulturmedium abgetrennt und der Überstand zur Bestimmung des in das Kulturmedium freigesetzten CD154-Fab-Fragments analysiert.

Die Quantifizierung des CD154-Fab-Fragments erfolgte über einen dem Fachmann bekannten Sandwich-ELISA-Test. Dabei diente ein immobilisierter Anti-human-IgG (Fd)-Antikörper (The Binding Site, Product No. PC075) als Fänger und ein Peroxidase-konjugierter goat Anti-Human Kappa Light Chain Antikörper (Sigma, Product No. A 7164) als Detektions-Antikörper. Die Quantifizierung erfolgte durch Umsetzung des chromogenen Substrats Dako TMB+ (Dako # S1599) durch die Peroxidase und die damit einhergehende Absorptionsänderung bei 450 nm. Zur Kalibrierung des ELISA wurde das Fab-Fragment "Human Fab/Kappa" (Bethyl Laboratories, Produktnummer: P80-115) verwendet.

**Tabelle 3:** Ausbeuten an CD154-Antikörper-Fragment im Kulturüberstand nach 48 h.

| Stamm | CD154-Antikörper Fragment (mg/l) im Überstand |
|---|---|
| W3110/pJF118ut-CD154 | 5 |
| W3110/pJF118ut-CD154-Spr | 1045 |

Um den Einfluss der Expression von Spr auf das Zellwachstum zu untersuchen, wurde zum Ende der Fermentation nach 48 h die Zelltrockenmasse der Kulturen wie in Beispiel 1 beschrieben bestimmt. Die Ergebnisse sind in Tabelle 4 zusammengefasst.

**Tabelle 4:** Zelltrockenmassen (CDW) der CD154-produzierenden Stämme im Fermenter nach 48 h.

| Stamm | CDW (g/l) |
|---|---|
| W3110/pJF118ut-CD154 | 48 |

**15**

(fortgesetzt)

| Stamm | CDW (g/l) |
|---|---|
| W3110/pJF118ut-CD154-Spr | 43 |

**Patentansprüche**

1. Verfahren zur fermentativen Produktion von rekombinanten Proteinen **dadurch gekennzeichnet, dass** ein Bakterienstamm enthaltend einen offenen Leserahmen codierend für ein Signalpeptid und ein rekombinantes Protein unter Kontrolle eines funktionellen Promotors,

   wobei der Bakterienstamm einen zusätzlichen offenen Leserahmen codierend für ein Signalpeptid und eine Peptidoglykan-Peptidase unter Kontrolle eines funktionellen Promotors enthält, wobei es sich bei der Peptidoglykan-Peptidase um (i) Spr, (ii) YdhO oder (iii) Spr und YdhO handelt, und wobei das als Peptidoglykan-Peptidase gewählte Protein Murein-DD-Endopeptidase-Aktivität aufweist,
   in einem Fermentationsmedium kultiviert, das Fermentationsmedium nach der Fermentation von den Zellen abgetrennt und das rekombinante Protein aus dem Fermentationsmedium isoliert wird,
   wobei das rekombinante Protein aus dem Periplasma der Zelle freigesetzt wird und die Ausbeute an rekombinantem Protein, das in das Kulturmedium freigesetzt wird, mindestens 1,1-mal so hoch ist wie die Ausbeute, die mit entsprechenden Bakterienstämmen ohne Überproduktion der Peptidoglykan-Peptidase erreicht werden kann, und
   wobei es sich bei dem Bakterienstamm um einen Stamm der Art *Escherichia coli* handelt.

2. Verfahren zur fermentativen Produktion von rekombinanten Proteinen gemäß Anspruch 1, **dadurch gekennzeichnet, dass** es sich bei Spr um die in SEQ ID Nr. 5 von Aminosäuren 27-188 angegebene Sequenz handelt.

3. Verfahren zur fermentativen Produktion von rekombinanten Proteinen gemäß einem oder mehreren der Ansprüche 1 bis 2, **dadurch gekennzeichnet, dass** es sich bei YdhO um die in SEQ ID Nr. 7 von Aminosäuren 28-271 angegebene Sequenz handelt.

4. Verfahren zur fermentativen Produktion von rekombinanten Proteinen gemäß einem oder mehreren der Ansprüche 1 bis 3, **dadurch gekennzeichnet, dass** es sich beim funktionellen Promotor, der die Expression des offenen Leserahmens codierend die Peptidoglykan-Peptidase kontrolliert, um einen induzierbaren Promotor handelt.

5. Verfahren zur fermentativen Produktion von rekombinanten Proteinen gemäß einem oder mehreren der Ansprüche 1 bis 4, **dadurch gekennzeichnet, dass** das rekombinante Protein ein heterologes Protein ist.

6. Verfahren gemäß einem oder mehreren der Ansprüche 1 bis 5, **dadurch gekennzeichnet, dass** die Expression der Peptidoglykan-Peptidase induziert wird.

7. Verfahren gemäß einem oder mehreren der Ansprüche 1 bis 6, **dadurch gekennzeichnet, dass** die Expression der Peptidoglykan-Peptidase nach der Induktion der Expression des rekombinanten Proteins induziert wird.

8. Verfahren gemäß einem oder mehreren der Ansprüche 1 bis 7, **dadurch gekennzeichnet, dass** sich 5 bis 24 Stunden nach Induktion der Expression der zusätzlichen Peptidoglykan-Peptidase die Zelltrockenmasse nach dem Abtrennen des Fermentationsmediums um höchstens 20% von der Zelltrockenmasse einer Zellkultur zum gleichen Zeitpunkt aus einem Fermentationsverfahren eines Bakterienstamms, der die zusätzliche Peptidoglykan-Peptidase nicht exprimiert, unterscheidet.


**Claims**

1. Method for fermentative production of recombinant proteins, **characterized in that** a bacterial strain containing an open reading frame encoding a signal peptide and a recombinant protein under the control of a functional promoter,

   wherein the bacterial strain contains an additional open reading frame encoding a signal peptide and a peptidoglycan peptidase under the control of a functional promoter, wherein the peptidoglycan peptidase is (i) Spr, (ii) YdhO or (iii) Spr and YdhO, and wherein the protein chosen as peptidoglycan peptidase has murein DD-endopeptidase activity,

is cultured in a fermentation medium, the fermentation medium is removed from the cells after the fermentation and the recombinant protein is isolated from the fermentation medium,

wherein the recombinant protein is released from the periplasm of the cell and the yield of recombinant protein which is released into the culture medium is at least 1.1 times as high as the yield which can be achieved with corresponding bacterial strains without overproduction of the peptidoglycan peptidase, and wherein the bacterial strain is a strain of the species *Escherichia coli*.

2. Method for fermentative production of recombinant proteins according to Claim 1, **characterized in that** Spr is the sequence specified by amino acids 27-188 in SEQ ID No. 5.

3. Method for fermentative production of recombinant proteins according to one or more of Claims 1 to 2, **characterized in that** YdhO is the sequence specified by amino acids 28-271 in SEQ ID No. 7.

4. Method for fermentative production of recombinant proteins according to one or more of Claims 1 to 3, **characterized in that** the functional promoter which controls the expression of the open reading frame encoding the peptidoglycan peptidase is an inducible promoter.

5. Method for fermentative production of recombinant proteins according to one or more of Claims 1 to 4, **characterized in that** the recombinant protein is a heterologous protein.

6. Method according to one or more of Claims 1 to 5, **characterized in that** the expression of the peptidoglycan peptidase is induced.

7. Method according to one or more of Claims 1 to 6, **characterized in that** the expression of the peptidoglycan peptidase is induced after the induction of the expression of the recombinant protein.

8. Method according to one or more of Claims 1 to 7, **characterized in that**, 5 to 24 hours after induction of the expression of the additional peptidoglycan peptidase, the cell dry weight after the removal of the fermentation medium differs by not more than 20% from the cell dry weight of a cell culture at the same time point from a fermentation method for a bacterial strain which does not express the additional peptidoglycan peptidase.

## Revendications

1. Procédé pour la production par fermentation de protéines recombinantes, **caractérisé en ce qu'**une souche bactérienne, contenant un cadre de lecture ouvert codant pour un peptide signal et une protéine recombinante sous le contrôle d'un promoteur fonctionnel,

la souche bactérienne contenant un cadre de lecture ouvert supplémentaire codant pour un peptide signal et une peptidoglycane-peptidase sous le contrôle d'un promoteur fonctionnel, la peptidoglycane-peptidase étant (i) Spr, (ii) YdhO ou (iii) Spr et YdhO, et la protéine choisie comme peptidoglycane-peptidase présentant une activité de muréine-DD-endopeptidase,

est cultivée dans un milieu de fermentation, après la fermentation, le milieu de fermentation est séparé des cellules et la protéine recombinante est isolée à partir du milieu de fermentation,

la protéine recombinante étant libérée à partir du périplasme de la cellule et le rendement en protéine recombinante, qui est libérée dans le milieu de culture, représentant au moins 1,1 fois le rendement qui peut être obtenu avec des souches bactériennes correspondantes sans surproduction de la peptidoglycane-peptidase et

la souche bactérienne étant une souche de l'espèce *Escherichia coli*.

2. Procédé pour la production par fermentation de protéines recombinantes selon la revendication 1, **caractérisé en ce qu'**il s'agit, pour Spr, de la séquence indiquée dans SEQ ID NO. 5 des acides aminés 27-188.

3. Procédé pour la production par fermentation de protéines recombinantes selon l'une ou plusieurs des revendications 1 à 2, **caractérisé en ce qu'**il s'agit, pour YdhO, de la séquence indiquée dans SEQ ID NO. 7 des acides aminés 28-271.

4. Procédé pour la production par fermentation de protéines recombinantes selon l'une ou plusieurs des revendications 1 à 3, **caractérisé en ce qu'**il s'agit, pour le promoteur fonctionnel qui contrôle l'expression du cadre de lecture ouvert codant pour la peptidoglycane-peptidase, d'un promoteur inductible.

5. Procédé pour la production par fermentation de protéines recombinantes selon l'une ou plusieurs des revendications 1 à 4, **caractérisé en ce que** la protéine recombinante est une protéine hétérologue.

6. Procédé selon l'une ou plusieurs des revendications 1 à 5, **caractérisé en ce que** l'expression de la peptidoglycane-peptidase est induite.

7. Procédé selon l'une ou plusieurs des revendications 1 à 6, **caractérisé en ce que** l'expression de la peptidoglycane-peptidase est induite après l'induction de l'expression de la protéine recombinante.

8. Procédé selon l'une ou plusieurs des revendications 1 à 7, **caractérisé en ce que** 5 à 24 heures après l'induction de l'expression de la peptidoglycane-peptidase supplémentaire, la masse sèche cellulaire après la séparation du milieu de fermentation se distingue d'au plus de 20% de la masse sèche cellulaire d'une culture cellulaire au même moment provenant d'un procédé de fermentation d'une souche bactérienne qui n'exprime pas la peptidoglycane-peptidase supplémentaire.

Fig. 1:

Fig. 2:

Fig. 3:

Fig. 4:

Fig. 5

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- CN 104762285 B
- EP 1124941 A
- WO 2013007388 A
- US 20080076157 A
- CN 104762285
- US 20080254511 A1
- US 2008076157 A

**In der Beschreibung aufgeführte Nicht-Patentliteratur**

- **CLAVEL et al.** *Mol. Microbiol.*, 1998, vol. 29, 359-367
- **CHEN et al.** *Microb. Biotechnol.*, 2014, vol. 7, 360-370
- **SILHAVY et al.** *Cold Spring Harb. Perspect. Biol.*, 2010, vol. 2 (5)
- **VOLLMER**. *FEMS Microbiol Rev*, 2008, vol. 32, 259-286
- **PIERCE et al.** *J Biotechnol*, 1997, vol. 58, 1-11
- **MORITA et al.** *Biotechnol. Prog.*, 2001, vol. 17, 573-576
- **SINGH et al.** *Mol. Microbiol.*, 2012, vol. 86, 1036-1051
- **YANG et al.** *Appl. Microbiol. Biotech*, 2018, vol. 103, 793-806
- **OJIMA et al.** *Biotech. Progr.*, 2017, vol. 34, S51-57
- **KLEINER-GROTE et al.** *Eng. Life Scie.*, 2018, vol. 18, 532-550
- **GONZALEZ-LEIZA et al.** *J Bacteriol*, 2011, vol. 193, 6887-94
- **CHOI** ; **LEE**. *Appl. Microbiol. Biotechnol.*, 2004, vol. 64, 625-635
- **KARPUSAS et al.** *Structure*, 2001, vol. 9, 321-329
- **MALACHOWSKA** ; **OLSZEWSKI**. *Microb Cell Fact*, 2018, vol. 17, 40